(19) Europäisches Patentamt
European Patent Office
Office européen des brevets

(11) Veröffentlichungsnummer: **0 308 371**
**A1**

# (12) EUROPÄISCHE PATENTANMELDUNG

(21) Anmeldenummer: **88810611.9**

(22) Anmeldetag: **09.09.88**

(51) Int. Cl.[4]: **C 07 D 513/04**
// A01N43/90

(30) Priorität: **18.09.87 CH 3620/87**

(43) Veröffentlichungstag der Anmeldung:
**22.03.89 Patentblatt 89/12**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Waldner, Adrian, Dr.**
**Holeeweg 39**
**CH-4123 Allschwil (CH)**

**Meyer, Willy**
**Talweg 49**
**CH-4125 Riehen (CH)**

(54) **4-Azasaccharine, 4-Aza-dihydro-oder-tetrahydrosaccharine und Verfahren zu deren Herstellung.**

(57) Verbindungen der Formel I

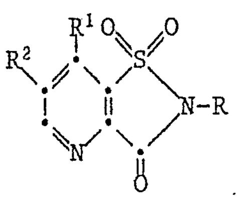

(I)

worin R H, ein aliphatischer oder aromatischer Rest bedeutet, $R^1$ Alkyl ist, $R^2$ Alkyl, Alkoxy, Aryl, Aryloxy, Aralkyl, Alkaryl, Aralkyloxy, Alkaryloxy, Alkaralkyl, Alkaralkyloxy darstellt, und eines von $R^1$ und $R^2$ H und $R^2$ bzw. $R^1$ die zuvor angegebene Bedeutung hat. Diese Verbindungen sind Zwischenprodukte zur Herstellung von herbiziden Sulfonylharnstoffen.

EP 0 308 371 A1

## Beschreibung

### 4-Azasaccharine, 4-Aza-dihydro- oder -tetrahydrosaccharine und Verfahren zu deren Herstellung

Die Erfindung betrifft mono- oder disubstituierte 4-Azasaccharine, mono-oder disubstituierte 4-Aza-dihydro- oder -tetrahydrosaccharine als Zwischenprodukte und Verfahren zu deren Herstellung.

R.F. Sauers et al. beschreiben in ACS Symposium Series 255, S. 21-28, (1984) Herbizide auf der Basis von Sulfonylharnstoffen. Herbizid wirksame Pyridylsulfonylharnstoffe sind beispielsweise in EP-A-0 013 480, EP-A-0 084 224, EP-A-0 097 122, EP-A-0 126 711 und EP-A-0 139 612 beschrieben. Die Mehrstufenverfahren zu deren Herstellung sind unwirtschaftlich und die Ausgangsprodukte sind schwer zugänglich.

In der EP-A-0172140 ist die Herstellung von Pyridin-2,3-dicarbonsäuren durch Diels-Alder-Reaktion von 1-Azadienen mit Ethylen-1,2-dicarbonsäurederivaten zu 1-Aminotetrahydropyridin-2,3-dicarbonsäurederivaten, Abspaltung der Aminogruppe und anschliessende Oxidation der erhaltenen 1,4-Dihydropyridine beschrieben.

Ein Gegenstand der Erfindung sind Verbindungen der Formel I

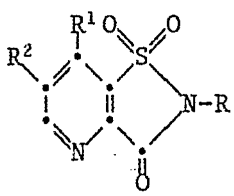

(I),

worin

R für H, lineares oder verzweigtes $C_1$-$C_{20}$-Alkyl, $C_2$-$C_{20}$-Alkenyl oder $C_2$-$C_{20}$-Alkinyl, $C_3$-$C_{10}$-Cycloalkyl, $C_4$-$C_{20}$-Cycloalkylalkyl, $C_4$-$C_{20}$-Alkylcycloalkyl, $C_5$-$C_{20}$-Alkylcycloalkylalkyl, $C_6$-$C_{14}$-Aryl, $C_7$-$C_{20}$-Aralkyl oder -Alkaryl oder $C_8$-$C_{20}$-Alkaralkyl steht, das unsubstituiert oder mit -OH, -CN, Halogen, $C_1$-$C_{12}$-Alkoxy oder -Alkylthio, $C_6$-$C_{10}$-Aryloxy oder -thio, $C_7$-$C_{16}$-Aralkyloxy, -Alkaryloxy, -Aralkylthio oder -Alkarylthio, $C_8$-$C_{18}$-Alkaralkyloxy oder -thio, $-NR^3R^4$, $-COOR^5$, $-OCOR^6$, $-CONR^3R^4$ oder $-NR^3COR^6$ substituiert ist, worin $R^3$ und $R^4$ unabhängig voneinander $C_1$-$C_{12}$-Alkyl, $C_5$- oder $C_6$-Cycloalkyl, $C_6$-$C_{10}$-Aryl, $C_7$-$C_{16}$-Aralkyl, $C_8$-$C_{16}$-Alkaralkyl oder $R^3$ und $R^4$ zusammen Tetra- oder Pentamethylen oder 3-Oxapentylen, $R^5$ H, $C_1$-$C_{12}$-Alkyl, Phenyl, Benzyl, Cyclohexyl oder Cyclopentyl und $R^6$ $C_1$-$C_{12}$-Acyl sind,

$R^1$ H, lineares oder verzweigtes $C_1$-$C_{12}$-Alkyl darstellt, das unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert ist,

$R^2$ H, $C_1$-$C_{12}$-Alkyl oder -Alkoxy, $C_6$-$C_{10}$-Aryl oder Aryloxy, $C_7$-$C_{16}$-Aralkyl, -Alkaryl, -Alkaryloxy oder -Aralkyloxy, $C_8$-$C_{16}$-Alkaralkyloxy bedeutet, die unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert sind.

R enthält als Alkyl bevorzugt 1-12, besonders 1-8 C-Atome. In einer bevorzugten Untergruppe steht R für $\alpha,\alpha$,-verzweigtes Alkyl mit besonders 4 bis 12 C-Atomen. Beispiele für Alkyl sind Methyl, Ethyl, n- und i-Propyl, n-, i- und t-Butyl, 1,1-Dimethylprop-1-yl, Pent-1-, -2- oder -3-yl, 1,1-, 1,2-, 1,3- oder 2,2-Dimethylprop-1-yl, 1,2,2-Trimethylprop-1-yl, 1-, 2-oder 3-Hexyl, 1,1-, 1,2-, 1,3-, 1,4-, 2,3- oder 3,4-Dimethylbut-1-yl, 1,1,2,2-Tetramethylethyl, 1-, 2-, 3-, oder 4-Heptyl, 1,1-Dimethylhex-1-yl, 2-Methylhept-2-yl, 3-Methylhept-3-yl, 1,1,2,2,-Tetramethylprop-1-yl, 1-, 2-, 3- oder 4-Octyl, 2-Ethyl-oct-1-yl, 1,1,3,3-Tetramethylbut-1-yl, 1-Methylhept-1-yl, 4-Methyl-hept-1-yl, sowie Nonyl, Decyl, Undecyl, Dodecyl, Tetradecyl, Hexadecyl, Heptadecyl, Octadecyl, Nonadecyl, Eicosyl und deren Isomere.

R enthält als Alkenyl und Alkinyl bevorzugt 2 bis 12, besonders 2-10 C-Atome. Diese Reste entsprechen besonders den Formeln $C_2$-$C_6$-Alkenyl-$C_nH_{2n}$- bzw. $C_2$-$C_6$-Alkinyl-$C_nH_{2n}$-, worin n bevorzugt für 1, 2 oder 3 steht. Beispiele sind Vinyl, Allyl, Crotonyl, But-2- oder But-3-en-1-yl, But-1-en-1-yl, But-1-en-3-yl, Pent-1-, -2- oder -3-en-5-yl, 1,1-Dimethylprop-2-en-1-yl, But-2-en-4-yl, Hex-1-, -2-, -3- oder -4-en-6-yl, Hex-2-, -3- oder 4-en-6-yl, Hex-2-en-4-yl, Hex-3-en-5- oder -6-yl, Hept-1-en-3-, -4-, -5-, -6- oder -7-yl, Hept-3-en-5- oder -6-yl, Oct-1-, -2-, -3-, -4-, -5-, oder -6-en-8-yl, Oct-2-en-4-yl, Non-7-en-9-yl, Dec-8-en-10-yl und entsprechende Alkinreste. Bevorzugt sind Allyl und Propargyl.

R enthält als Cycloalkyl bevorzugt 4 bis 8, besonders 5 oder 6 C-Atome. Beispiele sind Cyclopropyl, Cyclobutyl, Cycloheptyl, Cyclooctyl, Cyclononyl, Cyclodecyl und besonders Cyclopentyl oder Cyclohexyl.

R als Cycloalkylalkyl enthält bevorzugt 4-12-C-Atome und ist besonders $C_4$-$C_8$-Cycloalkyl-$C_nH_{2n}$-, worin n 1, 2 oder 3 ist. Das Cycloalkyl ist bevorzugt Cyclopentyl und Cyclohexyl. Beispiele sind Cyclopentylmethyl, 1- oder 2-Cyclopentyleth-1- oder -2-yl, Cyclohexylmethyl, 1- oder 2-Cyclohexyleth-1- oder -2-yl, 1-, 2- oder 3-Cyclohexylprop-1- oder -2-yl.

R als Alkylcycloalkyl enthält bevorzugt 4-12 C-Atome und ist besonders $C_1$-$C_9$-Alkyl-$C_4$-$C_8$-cycloalkyl. Das Cycloalkyl ist besonders Cyclopentyl oder Cyclohexyl. Beispiele sind Methyl- oder Dimethylcyclopentyl, Ethylcyclopentyl, Methyl- oder Dimethylcyclohexyl, Ethyl- oder Propyl-oder Butylcyclohexyl, Methylethylcyclohexyl.

R als Alkylcycloalkylalkyl enthält bevorzugt 5 bis 12 C-Atome und ist besonders $C_1$-$C_4$-Alkyl-$C_4$-$C_8$-Cycloalkyl-$C_nH_{2n}$-, worin n für 1, 2 oder 3 steht. Das Cycloalkyl ist bevorzugt Cyclopentyl oder Cyclohexyl. Beispiele sind Methylcyclohexylmethyl, 1- oder 2-(Methylcyclohexyl)-eth-1- oder 2-yl.

R enthält als Aryl bevorzugt 6-10 C-Atome. Beispiele sind Phenanthryl, Naphthyl und besonders Phenyl.

R enthält als Aralkyl bevorzugt 7-16, besonders 7-12 C-Atome. Das Aryl ist bevorzugt Phenyl. Das Aralkyl entspricht bevorzugt der Formel Phenyl-$C_nH_{2n}$-, worin n 1, 2 oder 3 ist. Beispiele sind Benzyl, 1- oder 2-Phenyleth-1- oder -2-yl, 1- oder 2-Phenylprop-1-, -2- oder -3-yl.

R enthält als Alkaryl bevorzugt 7 bis 16 C-Atome. Das Aryl ist bevorzugt Phenyl. R entspricht besonders $C_1$-$C_{10}$-Alkylphenyl. Beispiele sind Methylphenyl, Dimethylphenyl, Ethylphenyl, Methylethylphenyl, n- oder i-Propylphenyl, Butylphenyl, Hexylphenyl, Octylphenyl, Nonylphenyl, Decylphenyl.

Als Alkaralkyl enthält R bevorzugt 7 bis 16 C-Atome. Das Aryl ist bevorzugt Phenyl. Als Alkaralkyl ist R besonders $C_1$-$C_{16}$-Alkylphenyl-$C_nH_{2n}$, worin n 1, 2 oder 3 ist. Beispiele sind Methylbenzyl, Ethylbenzyl, n- oder i-Propylbenzyl, Butylbenzyl, Dimethylbenzyl, Octylbenzyl, Nonylbenzyl, (Methylphenyl)eth-1- oder -2-yl, (Methylphenyl)prop-1-, -2- oder -3-yl.

In einer bevorzugten Ausführungsform steht R für H oder unsubstituiertes oder substituiertes $C_1$-$C_{12}$-Alkyl, $C_6$-$C_{10}$-Aryl, $C_7$-$C_{16}$-Aralkyl oder -Alkaryl, oder $C_8$-$C_{16}$-Alkaralkyl, wobei das Aryl besonders Phenyl ist. In einer besonders bevorzugten Ausführungsform ist R H oder unsubstituiertes oder substituiertes $C_1$-$C_8$-Alkyl, Phenyl, Phenyl-$C_1$-$C_3$-alkyl, $C_1$-$C_4$-Alkylphenyl oder $C_1$-$C_4$-Alkylphenyl-$C_1$-$C_3$-alkyl.

Der Rest R kann ein- oder mehrfach, vorzugsweise ein- bis dreifach substituiert sein. Die Substituenten können sein:

-OH, -CN, Halogen, vorzugsweise Br, Cl, F;

$C_1$-$C_{12}$-, bevorzugt $C_1$-$C_4$-Alkoxy und -Alkylthio, z.B. Methoxy, Ethoxy, n-oder i-Propoxy, Butoxy, Methylthio, Ethylthio;

$C_6$-$C_{10}$-Aryloxy und -thio, besonders Phenoxy oder Phenylthio;

$C_7$-$C_{16}$-, bevorzugt $C_7$-$C_{12}$-Alkaryloxy oder -thio;

wobei es sich bei dem Arylrest bevorzugt um einen Phenylrest handelt, z.B. Methyl-, Dimethyl-, Ethyl, Methyl-ethyl-, n- oder i-Propyl-, Butyl-, Octyl- und Decylphenyloxy oder -thio;

$C_7$-$C_{16}$-, bevorzugt $C_7$-$C_{10}$-Aralkyloxy und -thio, wobei der Arylrest bevorzugt ein Phenylrest ist, z.B. Benzyloxy, Benzylthio, Phenylethyloxy;

$C_8$-$C_{16}$-, bevorzugt $C_8$-$C_{12}$-Alkaralkyloxy- und -thio, wobei der Arylrest bevorzugt ein Phenylrest ist, z.B. Methylbenzyloxy, Ethylbenzyloxy, (Methylphenyl)ethyloxy, Methylbenzylthio;

-$NR^3R^4$, -$CONR^3R^4$ und -$NR^3COR^6$, worin $R^3$ und $R^4$ unabhängig voneinander bevorzugt $C_1$-$C_4$-Alkyl, Cyclopentyl, Cyclohexyl, Phenyl oder Benzyl sind und $R^6$ bevorzugt $C_1$-$C_8$-Acyl ist. Beispiele für Acyl sind Formyl, Acetyl, Propionyl, Butanoyl, Hexanoyl, Benzoyl, Chloracetyl, Dichloracetyl, Fluoracetyl, Trichloracetyl und Trifluoracetyl;

-$COOR^5$ und -$OCOR^6$, worin $R^5$ bevorzugt H oder $C_1$-$C_4$-Alkyl ist, wobei für $R^6$ die zuvor gegebenen Bevorzugungen gelten.

Die Substituenten Alkoxy, Alkylthio, Aryloxy, Arylthio, Alkaryloxy und -thio, Aralkyloxy und -thio, Alkaralkyloxy und -thio können ihrerseits substituiert sein, z.B. mit -OH, -CN, F, Cl, $C_1$-$C_4$-Alkoxy, -$NR^3R^4$, -$COOR^5$, -$OCOR^6$, -$CONR^3R^4$ oder -$NR^3COR^6$, wobei für $R^3$ bis $R^6$ die zuvor angegebenen Bedeutungen und Bevorzugungen gelten. Beispiele für solche Substituenten sind β-Chlorethoxy, β-Cyanoethoxy, Chlorphenoxy, Dichlorphenoxy, Fluorphenoxy, Trifluorphenoxy, Chlortrifluormethylphenoxy, Cyanophenyl, Chlorbenzyloxy, (Methoxycarbonyl)ethoxy oder -methoxy, Acetyloxyethoxy oder -methoxy, Dimethylaminocarbonylphenoxy.

$R^1$ enthält als Alkyl bevorzugt 1 bis 8, besonders 1 bis 4 C-Atome. Bevorzugte Beispiele sind Methyl, Ethyl, n- und i-Propyl. In einer bevorzugten Ausführungsform ist $R^1$ H. Bevorzugte Substituenten für $R^1$ sind Hydroxy, Cyano, F, Cl, Carboxyl und $C_1$-$C_4$-Alkoxy.

$R^2$ enthält als Alkyl und Alkoxy bevorzugt 1 bis 6 C-Atome und ist z.B. Methyl, Ethyl, n- und i-Propyl, n- und i-Butyl, Pentyl, Hexyl, Methoxy, Ethoxy, n- oder i-Propoxy, n- oder i-Butoxy. Als Aryl ist $R^2$ besonders Phenyl und als Aryloxy besonders Phenoxy. $R^2$ als Aralkyl bzw. Aralkyloxy ist bevorzugt Phenyl-$C_nH_{2n}$ $\leftarrow O \rightarrow_m$, worin n 1, 2 oder 3 und m 0 oder 1 ist; bevorzugt sind Benzyl und Benzyloxy. $R^2$ als Alkaryl oder Alkaryloxy ist bevorzugt $C_1$-$C_4$-Alkylphenyl oder -phenoxy, z.B. Methylphenyl, Dimethylphenyl, Methylphenoxy. $R^2$ als Alkaralkyl oder Alkaralkyloxy ist bevorzugt $C_1$-$C_4$-Alkylbenzyl oder -benzyloxy, z.B. Methylbenzyl oder Methylbenzyloxy.

In einer bevorzugten Untergruppe ist $R^2$ unsubstituiertes oder substituiertes $C_1$-$C_6$-Alkyl oder -Alkoxy, Phenyl, Phenyloxy, Benzyl, Benzyloxy, $C_1$-$C_4$-Alkylphenyl oder -Alkylphenoxy oder $C_1$-$C_4$-Alkylbenzyl oder -benzyloxy.

Von den Verbindungen der Formel I sind diejenigen bevorzugt, in welchen R H, α,α-verzweigtes $C_4$-$C_{12}$Alkyl, oder unsubstituiertes oder durch Methoxy, Nitro oder Halogen substituiertes Benzyl, $R^1$ H oder $C_1$-$C_4$Alkyl und $R^2$ H, $C_1$-$C_4$Alkyl oder $C_1$-$C_4$Alkoxy bedeutet. Von diesen bevorzugten Verbindungen der Formel I sind wiederum diejenigen, in welchen R H oder α,α-verzweigtes $C_4$-$C_8$Alkyl und insbesondere diejenigen in welchen R H bedeutet, besonders bevorzugt.

Die Verbindungen der Formel I können analog zu dem in der EP-A-0 172 140 beschriebenen Verfahren hergestellt werden, bei dem man ein Azadien der Formel II

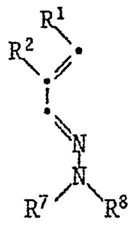

(II),

worin $R^7$ und $R^8$ unabhängig die Bedeutung von $R^3$ und $R^4$ haben, mit einer Verbindung der Formel III

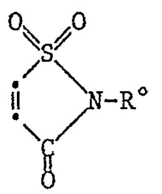

(III),

worin $R^0$ die Bedeutung von R, ausgenommen H hat, zu einer Verbindung der Formel IV

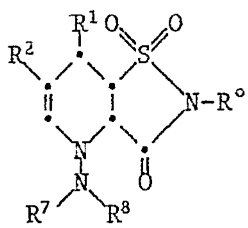

(IV)

umsetzt, die Verbindung der Formel IV durch Behandeln mit einer Säure und/oder durch thermische Behandlung unter Abspaltung von $R^7R^8NH$ in eine Verbindung der Formel V überführt

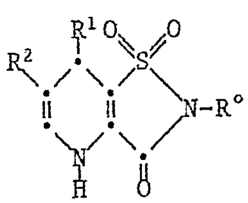

(V),

die Verbindung der Formel V zu einer Verbindung der Formel I oxidiert, und zur Herstellung von Verbindungen der Formel I worin R H bedeutet, die Verbindung der Formel V mit einer starken Säure umsetzt.

Die Verfahrensbedingungen sind im einzelnen in der EP-A-0 172 140 beschrieben.

Für die Abspaltung der Gruppe $R^0$ zur Herstellung von Verbindungen der Formel I mit R = H verwendet man bevorzugt halogenierte aliphatische $C_2$-$C_4$-Carbonsäuren, z.B. Difluor- oder Trifluoressigsäure, $\alpha,\alpha$-Difluorpropionsäure, Perfluorpropionsäure oder 1,1,1-Trifluor-2,2-dichlorpropionsäure.

Die ungesättigten Hydrazone der Formel II sind bekannt oder können durch Umsetzung von $\alpha,\beta$-ungesättigten Aldehyden mit N,N-substituierten Hydrazinen hergestellt werden. Die Verbindungen der Formel III sind z.B. in J. of Heteroc. Chem., 8, 571 und 591 (1971) beschrieben oder können analog nach den dort beschriebenen Verfahren hergestellt werden.

Ein weiterer Gegenstand der Erfindung sind die Verbindungen der Formeln IV und V in denen $R^0$ die Bedeutung von R hat, ausgenommen H. Für R, $R^1$ und $R^2$ gelten die für die Verbindung der Formel I gegebenen Bevorzugungen.

Es wurde gefunden, dass man dann direkt zu bestimmten Verbindungen der Formel I gelangt, wenn $R^0$ in

Formel III ein α,α-verzweigter aliphatischer Rest ist und die Reaktion in einem organischen Sulfoxid als Lösungsmittel durchgeführt wird.

Ein weiterer Gegenstand der Erfindung ist ein Verfahren zur Herstellung von Verbindungen der Formel Ia

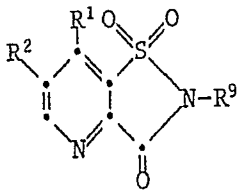

(Ia),

worin $R^1$ und $R^2$ die zuvor angegebene Bedeutung haben und $R^9$ α,α-verzweigtes $C_4$-$C_{20}$-Alkyl, $C_5$-$C_{20}$-Alkenyl oder -Alkinyl, 1-($C_1$-$C_4$-Alkyl)-$C_3$-$C_{10}$-Cycloalk-1-yl, α,α-verzweigtes $C_6$-$C_{20}$-Cycloalkylalkyl, $C_7$-$C_{20}$-Alkylcycloalkylalkyl, $C_9$-$C_{16}$-Aralkyl oder $C_{10}$-$C_{16}$-Alkaralkyl bedeutet, das dadurch gekennzeichnet ist, dass man eine Verbindung der Formel II

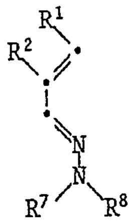

(II),

worin $R^7$ und $R^8$ unabhängig die Bedeutung von $R^3$ und $R^4$ haben, mit einer Verbindung der Formel IIIa

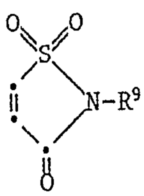

(IIIa),

worin $R^9$ die zuvor angegebene Bedeutung hat,in Gegenwart von Sauerstoff und einem organischen Sulfoxid als Lösungsmittel bei erhöhter Temperatur unter Abspaltung von $R^7R^8NH$ umsetzt.

$R^9$ kann wie für R in Formel I definiert substituiert sein.

$R^9$ ist insbesondere α,α-verzweigtes $C_4$-$C_{12}$-Alkyl. $R^9$ als α,α-verzweigtes Alkenyl oder Alkinyl enthält bevorzugt 5-10 C-Atome. $R^9$ als 1-($C_1$-$C_4$-Alkyl)cycloalk-1-yl enthält bevorzugt 4 bis 8 besonders 5 oder 6 C-Atome im Cycloalkylrest. $R^9$ als α,α-verzweigtes Cycloalkylalkyl ist bevorzugt $C_4$-$C_8$-, besonders $C_5$-oder $C_6$-Cycloalkyl-$C_3$-$C_8$-alkyl. $R^9$ als, α,α-verzweigtes Alkylcycloalkylalkyl ist bevorzugt $C_1$-$C_4$-Alkyl-$C_4$-$C_8$-, besonders -$C_5$- oder -$C_6$-cycloalkyl-$C_3$-$C_8$-, besonders -$C_3$-$C_6$-Alkyl. $R^9$ als α,α-verzweigtes Aralkyl enthält besonders 9 bis 14 C-Atome und stellt besonders Phenyl-$C_3$-$C_6$-Alkyl dar. Als Alkaralkyl ist $R^9$ bevorzugt $C_1$-$C_4$-Alkylphenyl-$C_3$-$C_6$-alkyl. Beispiele sind t-Butyl, 1,1-Dimethyl-but-1-yl oder -prop-1-yl, 2-Methyl-but-2-yl, 1,1,3,3-Tetramethylprop-1-yl, 1,1,2,2-Tetramethylethyl, 1,1-Dimethylpent-1-yl oder -hex-1-yl, 1-Methylcyclohex-1-yl, 2-Cyclopentylprop-2-yl, 2-(Methylcyclohexyl)prop-2-yl, 2-Phenylprop-2-yl und 2-(Methylphenyl)-prop-2-yl.

Geeignete Sulfoxide sind z.B. Diethylsulfoxid, Methylethylsulfoxid, Tetramethylen- oder Pentamethylensulfoxid und besonders Dimethylsulfoxid.

Die Reaktionstemperatur beträgt bevorzugt 50 bis 200°C, besonders 60 bis 150°C. Es kann unter reiner Sauerstoffatmosphäre oder Sauerstoff/Inergas-Gemischen gearbeitet, z.B. Luft.

Es wurde ferner gefunden, dass bei Verwendung eines organischen Sulfoxids als Lösungsmittel Verbindungen der Formel I in einem Zweistufenverfahren erhalten werden, wobei unter Abspaltung von $R^7R^8NH$ in der ersten Stufe der Pyridinring gebildet wird.

Ein weiterer Gegenstand der Erfindung ist ein Verfahren zur Herstellung von Verbindungen der Formel Ib

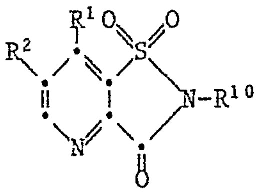

(Ib),

worin $R^1$ und $R^2$ die zuvor angegebene Bedeutung haben und $R^{10}$ die Bedeutung von R hat, aber nicht H ist und nicht die Bedeutung von $R^9$ hat, das dadurch gekennzeichnet ist, dass man eine Verbindung der Formel II

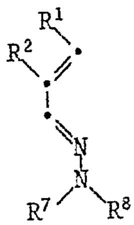

(II),

worin $R^7$ und $R^8$ unabhängig die Bedeutung von $R^3$ und $R^4$ haben, in Gegenwart von Sauerstoff und einem organischen Sulfoxid als Lösungsmittel bei erhöhten Temperaturen mit einer Verbindung der Formel IIIb

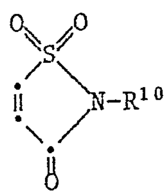

(IIIb),

zu einer Verbindung der Formel VI

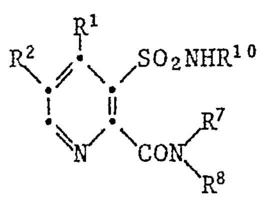

(VI)

umsetzt, und die Verbindung VI unter Abspaltung von $R^7R^8NH$ zu einer Verbindung der Formel Ib cyclisiert.

Für $R^{10}$ gelten die gleichen Bevorzugungen wie für R in Formel I und für die Reaktionsbedingungen die Bevorzugungen für das zuvor beschriebene Verfahren.

Die Cyclisierung wir bevorzugt mit starken Säuren, z.B. konzentrierter Schwefelsäure durchgeführt. Vorteilhaft wird Eisessig als Lösungsmittel verwendet.

Die Isolierung der Verbindungen der Formeln I, Ia, Ib, IV, V und VI kann in üblicher Weise erfolgen, z.B. durch Abdampfen des Lösungsmittels und Filtrieren, Sublimieren oder Destillieren. Die Verbindungen können durch Umkristallisation oder chromatographische Methoden weiter gereinigt werden.

Die Verbindungen der Formel I sind wertvolle Zwischenprodukte, z.B. zur Herstellung von Sulfonylharnstoffen, die als Herbizide verwendet werden können (vgl. R.F. Sauers et al., ACS Symposium Series 255, S. 21-28 (1984)).

Die nachfolgenden Beispiele erläutern die Erfindungen näher. Prozente sind Gewichtsprozente, sofern nicht anders angegeben. Der Nomenklatur liegt folgende Strukturformel des 4-Azasaccharins zu Grunde:

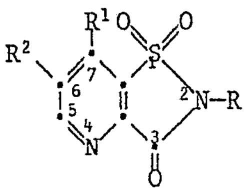

Herstellungsbeispiele:

Beispiel 1:

18,9 g 2-Ethyl-4-dimethylamino-4-azabutadien, und 12,6 g 2-t-Butyl-isothiazolin-3-on-1,1-dioxid werden in 200 ml Ethanol 8 Stunden am Rückfluss erhitzt. Nach dem Eindampfen wird der Rückstand aus Ether/Petrolether umkristallisiert. Man erhält 25 g (79 % der Theorie) kristalline Verbindung der Formel IVa

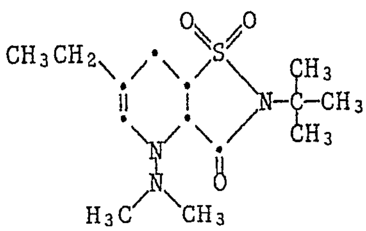

IVa

Beispiel 2:

a) 6,4 g 2-Methoxy-acrolein werden bei 30° - 35° C mit 4,4 g N,N-Dimethylhydrazin in 50 ml Aether 1 Std. gerührt, bei 20°C mit $Na_2SO_4$ getrocknet und das Lösungsmittel entfernt. Durch Destillation bei 74° - 76° C/13 mbar 6,2 g reines 2-Methoxy-4-Dimethylamino-4-azabutadien.

b) 10,8 g 2-Methoxy-4-Dimethylamino-4-azabutadien und 15 g 2-t-Butyl-isothiazolin-3-on-1,1-dioxid werden in 250 ml Toluol 1 1/2 h bei 80° - 85°C gerührt. Der rohe Bicyclus der Formel IV b wird wie in Beispiel 4 beschrieben, roh weiter verarbeitet.

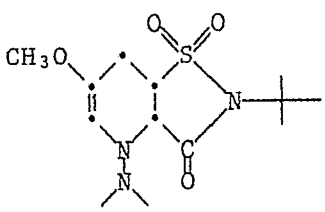

IVb

Auf analoge Weise erhält man die in Tabelle 1 aufgeführten Verbindungen.

Tabelle 1

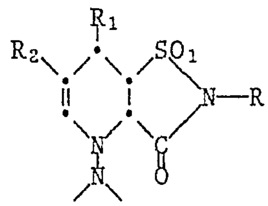

| Nr. | $R_1$ | $R_2$ | R | Smp. |
|---|---|---|---|---|
| 01.01 | H | $CH_2CH_3$ | t-Butyl | 90° - 92°C |
| 01.02 | $CH_3$ | $CH_3$ | t-Butyl | 50° - 52°C |
| 01.03 | H | $CH_3$ | t-Butyl | |
| 01.04 | H | $CH_2CH_3$ | $-C(CH_3)_2-CH_2C(CH_3)_3$ | |
| 01.05 | H | $OCH_3$ | t-Butyl | |
| 01.06 | H | $OCH_2$— 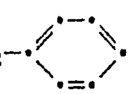 | t-Butyl | |
| 01.07 | H | $OCH_2CH_3$ | t-Butyl | |
| 01.08 | $CH_3$ | H | t-Butyl | |
| 01.09 | $CH_3$ | $OCH_3$ | t-Butyl | |
| 01.10 | H | $OCH_3$ | $CH_2$— 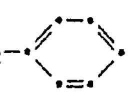 | |
| 01.11 | H | $CH_2OCH_3$ | t-Butyl | |
| 01.12 | H | $SCH_3$ | t-Butyl | |
| 01.13 | H | $CH(CH_3)_2$ | t-Butyl | |

Beispiel 3:

25 g Cycloaddukt der Struktur gemäss Beispiel 1 und 55 g Kieselgel werden in 300 ml Toluol 75 Minuten auf 100° C erhitzt. Nach dem Abfiltrieren und Auswaschen des Kieselgels wird die gelbe Lösung eingedampft und der Rückstand mit Petrolether digeriert. Man erhält 15,5 g (73 %) gelbes Pulver, das aus $CCl_4$ umkristallisiert wird. Analog werden anders substituierte Cycloaddukte umgesetzt. Man erhält

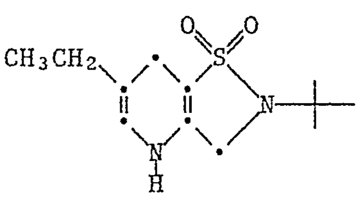 :

Beispiel 4:
Das Cycloaddukt gemäss Beispiel 2 wird bei R.T. mit 9 gr Trifluoressigsäure versetzt und 1 Std. nachgerührt. Der rohe Bicyclus der Formel Va wird wie in Beispiel 6 beschrieben weiterverarbeitet.

Auf analoge Weise erhält man die in Tabelle 2 aufgeführten Verbindungen.

Tabelle 2

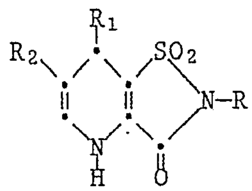

| Nr. | $R^1$ | $R^2$ | R | Smp. |
|---|---|---|---|---|
| 02.01 | H | $CH_2CH_3$ | t-Butyl | 161° - 163°C |
| 02.02 | $CH_3$ | $CH_3$ | t-Butyl | 133° - 136°C |
| 02.03 | H | $CH_3$ | t-Butyl | 178° - 179°C |
| 02.04 | H | $CH_2CH_3$ | $C(CH_3)_2CH_2C(CH_3)_3$ | 109° - 113°C |
| 02.05 | H | $OCH_3$ | t-Butyl | |
| 02.06 | H | $OCH_2$— 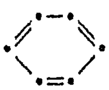 | t-Butyl | |
| 02.07 | H | $OCH_2CH_3$ | t-Butyl | |
| 02.08 | $CH_3$ | H | t-Butyl | |
| 02.09 | $CH_3$ | $OCH_3$ | t-Butyl | |
| 02.10 | H | $OCH_3$ | $CH_2$— 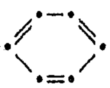 | |
| 02.11 | H | $CH_2OCH_3$ | t-Butyl | |
| 02.12 | H | $SCH_3$ | t-Butyl | |
| 02.13 | H | $CH(CH_3)_2$ | t-Butyl | |

Beispiel 5:
14,1 g Verbindung mit der in Beispiel 3 angegebenen Struktur werden in 130 ml Eisessig auf 65° C erwärmt und mit 6,4 g Braunstein versetzt. Nach einer Stunde bei 60° C wird filtriert und eingedampft. Der Rückstand wird zwischen Wasser und Chloroform verteilt. Die organische Phase wird zweimal mit Wasser gewaschen, getrocknet und eingedampft. Der hallbeige Rückstand wird mit Petrolether digeriert. Man erhält 13 g (93 %) 6-Aethyl-N-t-butyl-4-azasacharin.

Beispiel 6:
Aequimolare Mengen 2-Methyl-4-dimethylamino-4-azabutadien und 2-t-Butylisothiazolin-3-on-2,2-dioxid werden in Dimethylsulfoxid auf 80 bis 90° C erhitzt. Nach 1 Stunde wird auf 100° C Innentemperatur erhitzt. Nach einer Induktionsphase tritt Exothermie ein. Das Heizbad wird entfernt und mit einem Wasserbad gekühlt. Es wird noch 30 Minuten bei 100° C gerüht. Danach wird das Lösungsmittel im Hochvakuum abdestilliert und der Rückstand aus $CCl_4$ umkristallisiert. Man erhält 40 % Ausbeute an 6-Methyl-N-t-butyl-4-azasacharin.

Beispiel 7:
Das Cycloaddukt gemäss Beispiel 4 wird bei R.T. mit 12,6 g Brom versetzt. Anschliessend tropft man 24 g Triäthylamin zu und rührt 15 Std. bei 20° - 25° C nach. Die Suspension wird total eingedampft, mit Wasser und

Essigester versetzt und die organische Phase abgetrennt. Diese wird total eingedampft und der Rückstand aus Aethanol umkristallisiert. Auf diese Art erhält man die Verbindung der Formel Ic mit einem Schmelzpunkt von 216° - 217°C

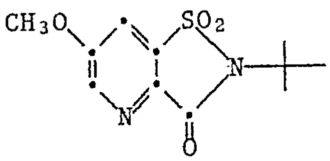

Ic

Tabelle 3

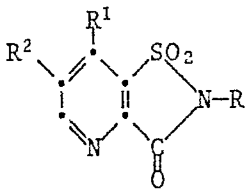

| Nr. | $R^1$ | $R^2$ | R | Smp. |
|---|---|---|---|---|
| 03.01 | H | $CH_2CH_3$ | t-Butyl | 149° - 151°C |
| 03.02 | $CH_3$ | $CH_3$ | t-Butyl | 150° - 152°C |
| 03.03 | H | $CH_3$ | t-Butyl | 170° - 172°C |
| 03.04 | H | $CH_3$ | $C(CH_3)_2CH_2C(CH_3)_3$ | 221° - 222°C |
| 03.05 | H | $OCH_3$ | t-Butyl | 216° - 217°C |
| 03. 06 | H | $OCH_2$— 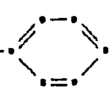 | t-Butyl | |
| 03.07 | H | $OCH_2CH_3$ | t-Butyl | |
| 03.08 | $CH_3$ | H | t-Butyl | |
| 03.09 | $CH_3$ | $OCH_3$ | t-Butyl | |
| 03.10 | H | $OCH_3$ | $CH_2$— 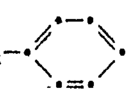 | |
| 03.11 | H | $CH_2OCH_3$ | t-Butyl | |
| 03.12 | H | $SCH_3$ | t-Butyl | |
| 03.13 | H | $CH(CH_3)_2$ | t-Butyl | 123° - 125°C |

Beispiel 8:

11,5 g Verbindung gemäss Beispiel 7 wird in 42 ml Trifluoressigsäure auf 120°C erhitzt. Nach 3 Stunden wird eingedampft und der Rückstand mit Essigsäureethylester/Diethylether digeriert. Das Pulver wird abfiltriert und im Vakuum getrocknet. Man erhält das 6-Aethylazasacharin in einer Ausbeute von 60 % und einem Schmelzpunkt von 199° - 201°C.

Tabelle 4:

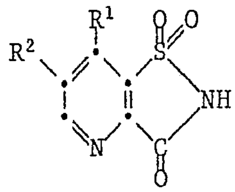

| Nr. | $R^1$ | $R^2$ | Smp. |
|---|---|---|---|
| 04.01 | H | $CH_2CH_3$ | 199° - 201°C |
| 04.02 | $CH_3$ | $CH_3$ | 267° - 270°C |
| 04.03 | H | $CH_3$ | 215° - 217°C |
| 04.04 | H | $OCH_3$ | 255° - 256°C |
| 04.05 | H | $OCH_2$— 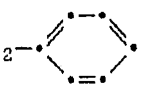 | |
| 04.06 | H | $OCH_2CH_3$ | |
| 04.07 | $CH_3$ | H | |
| 04.08 | $CH_3$ | $OCH_3$ | |
| 04.09 | H | $CH_2OCH_3$ | |
| 04.10 | H | $CH(CH_3)_2$ | 199° - 202°C |

Beispiel 9:

Es wird wie in Beispiel 6 verfahren und 2-Methyl-4-dimethylamino-4-azabutadien mit einem Isothiazolin umgesetzt, dessen N-Atom durch Benzyl bzw. p-Methoxybenzyl substituiert ist. Man erhält.

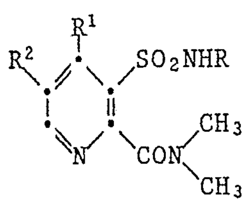

:

| Beispiel No. | $R^1$ | $R^2$ | R | Schmelzpunkt (°C) | Ausbeute (%) |
|---|---|---|---|---|---|
| 10 | H | $CH_3$ | Benzyl | 169-70 | 43 |
| 11 | H | $CH_3$ | p-Methoxybenzyl | 141-3 | 42 |

Beispiel 12:

5,0 g Verbindung gemäss Beispiel 10 werden in 30 ml Eisessig und 0,75 g konzentrierter Schwefelsäure 20 Stunden auf 100°C erhitzt. Das Reationsgemisch wird eingedampft, der Rückstand in Chloroform gelöst und mit 1n $NaHCO_3$-Lösung gewaschen. Die organische Phase wird über $MgSO_4$ getrocknet und dann eingedampft. Man digeriert mit Diethylether und erhält 3,9 g (90 %) 6-Methyl-N-benzyl-4-azasaccharin als beige Kristalle vom Schmelzpunkt 167-70°C.

Anwendungsbeispiel

a) Herstellung eines Sulfonylharnstoffes

1 Mol 6-Methyl-4-azasaccharin wird im 10-fachen Volumen Methanol suspendiert und mit $\frac{1}{10}$ Mol-Aequivalent konzentrierter Schwefelsäure im Bombenrohr auf 100°C erhitzt. Nach 18 Stunden wird eingedampft und der Rückstand mit $CHCl_3$/$NaHCO_3$ gewaschen. Man erhält 3-(Aminosulfonyl)-5-methylpyridin-2-carbonsäuremethylester als beiges Pulver vom Schmelzpunkt 86-8°C.

Man löst 1 Mol des Esters in Acetonitril und tropft während 75 Minuten eine Lösung von 5-Methyl-3-methoxy-1-(phenoxycarbonylamino)triazin in Acetonitril zu. Nach einer Stunde Rühren wird eingedampft, mit Wasser aufgenommen und 1 Moläquivalent 1n HCl zugetropft. Der Niederschlag wird abfiltriert und getrocknet. Man erhält

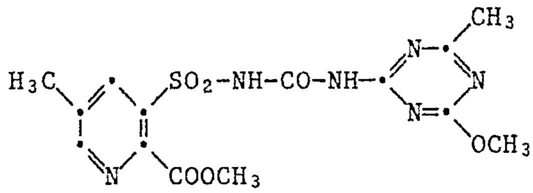

vom Schmelzpunkt 168-70°C.

b) Nachweis der herbiziden Wirkung bei Vorlaufanwendung

Im Gewächshaus werden Pflanzensamen von dikotylen und monokotylen Unkräutern in Töpfe von 11 cm Durchmesser gesät. Unmittelbar danach wird die Erdoberfläche mit einer wässrigen Dispersion oder Lösung der Wirkstoffe behandelt. Es werden Konzentrationen von 0,5, 0,125 und 0,03 kg Wirkstoffmengen pro Hektar angewendet. Die Töpfe werden dann im Gewächshaus bei einer Temperatur von 22-25°C und 50-70 % relativer Luftfeuchtigkeit gehalten. Nach 3 Wochen wird der Versuch ausgewertet und die Wirkung nach folgendem Massstab beurteilt:

1 Pflanze nicht gekeimt oder abgestorben
2-3 sehr starke Wirkung
4-6 mittlere Wirkung
7-8 schwache Wirkung
9 keine Wirkung (wie unbehandelte Kontrolle).

Versuchsergebnisse (pre-emergent)

| Testpflanze | Wirkung Aufwandmenge kg/ha | | |
|---|---|---|---|
| | 0,500 | 0,125 | 0,03 |
| Alopecurus myos. | 3 | 4 | 5 |
| Echinochloa c.g. | 2 | 3 | 7 |
| Amaranthus ret. | 2 | 2 | 3 |
| Chenopodium Sp. | 3 | 4 | 7 |
| Sinapsis | 2 | 2 | 2 |
| Stellaria | 3 | 4 | 4 |
| Chrysanth. leuc. | 2 | 2 | 3 |
| Galium aparine | 2 | 3 | 5 |
| Viola tricolor | 2 | 2 | 2 |
| Veronica Sp | 2 | 3 | 3 |

**Patentansprüche**

1. Verbindungen der Formel I

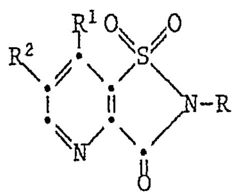

(I),

worin
R für H, lineares oder verzweigtes $C_1$-$C_{20}$-Alkyl, $C_2$-$C_{20}$-Alkenyl oder $C_2$-$C_{20}$-Alkinyl, $C_3$-$C_{10}$-Cycloalkyl, $C_4$-$C_{20}$-Cycloalkylalkyl, $C_4$-$C_{20}$-Alkylcycloalkyl, $C_5$-$C_{20}$-Alkylcycloalkylalkyl, $C_6$-$C_{14}$-Aryl, $C_7$-$C_{20}$-Aralkyl oder -Alkaryl oder $C_8$-$C_{20}$-Alkaralykl steht, das unsubstituiert oder mit -OH, -CN, Halogen, $C_1$-$C_{12}$-Alkoxy oder -Alkylthio, $C_6$-$C_{10}$-Aryloxy oder -thio, $C_7$-$C_{16}$-Aralkyloxy, -Alkaryloxy, -Aralkylthio oder -Alkarylthio, $C_8$-$C_{18}$-Alkaralkyloxy oder -thio, -$NR^3$-$R^4$, -$COOR^5$, -$OCOR^6$, -$CONR^3R^4$ oder -$NR^3$-$COR^6$ substituiert ist, worin $R^3$ und $R^4$ unabhängig voneinander $C_1$-$C_{12}$-Alkyl, $C_5$- oder $C_6$-cycloalkyl, $C_6$-$C_{10}$-Aryl, $C_7$-$C_{16}$-Aralkyl, $C_8$-$C_{16}$-Alkaralkyl oder $R^3$ und $R^4$ zusammen Tetra- oder Pentamethylen oder 3-Oxapentylen, $R^5$ H, $C_1$-$C_{12}$-Alkyl, Phenyl, Benzyl, Cyclohexyl oder Cyclopentyl und $R^6$ $C_1$-$C_{12}$-Acyl sind, $R^1$ H, lineares oder verzweigtes $C_1$-$C_{12}$-Alkyl darstellt, das unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert ist, $R^2$ H, $C_1$-$C_{12}$-Alkyl oder -Alkoxy, $C_6$-$C_{10}$-Aryl oder -Aryloxy, $C_7$-$C_{16}$-Aralkyl, -Alkaryl, -Alkaryloxy oder -Aralkyloxy, $C_8$-$C_{16}$-Alkaralkyloxy bedeutet, die unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert sind.

2. Verbindungen der Formel I gemäss Anspruch 1, worin R für H, α,α-verzweigtes $C_4$-$C_{12}$Alkyl oder für unsubstituiertes oder durch Methoxy, Nitro oder Halogen substituiertes Benzyl steht, $R^1$ H oder $C_1$-$C_4$Alkyl bedeutet und $R^2$ H, $C_1$-$C_4$Alkyl oder $C_1$-$C_4$Alkoxy darstellt.

3. Verbindungen der Formel I gemäss Anspruch 2 worin R für H oder α,α-verzweigtes $C_4$-$C_8$Alkyl steht.

4. Verbindungen der Formel I gemäss Anspruch 2, worin R für H steht.

5. Verfahren zur Herstellung von Verbindungen der Formel I gemäss Anspruch 1, bei dem man ein Azadien der Formel II

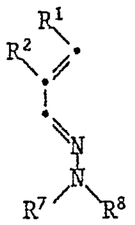

(II),

worin $R^7$ und $R^8$ unabhängig die Bedeutung von $R^3$ und $R^4$ haben, mit einer Verbindung der Formel III

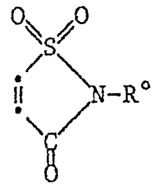

(III),

worin $R^0$ die Bedeutung von R, ausgenommen H hat, zu einer Verbindung der Formel IV

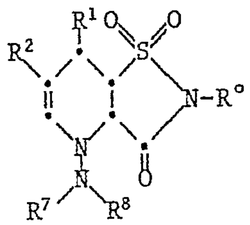

(IV)

umsetzt, die Verbindung der Formel IV durch Behandeln mit einer Säure und/oder durch thermische Behandlung unter Abspaltung von $R^7R^8NH$ in eine Verbindung der Formel V überführt

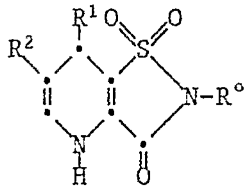

(V),

die Verbindung der Formel V zu einer Verbindung der Formel I oxidiert, und zur Herstellung von Verbindungen der Formel I worin R H bedeutet, die Verbindung der Formel V mit einer starken Säure umsetzt.

6. Verfahren zur Herstellung von Verbindungen der Formel Ia

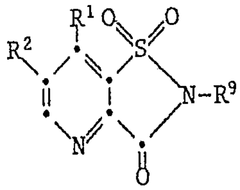

(Ia),

worin $R^1$ und $R^2$ die im Anspruch 1 gegebene Bedeutung haben und $R^9$ α,α-verzweigtes $C_4$-$C_{20}$-Alkyl, $C_5$-$C_{20}$-Alkenyl oder -Alkinyl, 1-($C_1$-$C_4$-Alkyl)-$C_3$-$C_{10}$-Cycloalk-1-yl, α,α-verzweigtes $C_6$-$C_{20}$-Cycloalkylalkyl, $C_7$-$C_{20}$-Alkylcycloalkylalkyl, $C_9$-$C_{16}$-Aralkyl oder $C_{10}$-$C_{16}$-Alkaralkyl bedeutet, dadurch gekennzeichnet, dass man eine Verbindung der Formel II

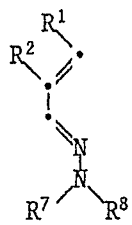

(II),

worin $R^7$ und $R^8$ unabhängig die Bedeutung von $R^3$ und $R^4$ haben, mit einer Verbindung der Formel IIIa

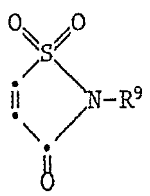

(IIIa),

worin $R^9$ die zuvor angegebene Bedeutung hat, in Gegenwart von Sauerstoff und einem organischen Sulfoxid als Lösungsmittel bei erhöhter Temperatur unter Abspaltung von $R^7R^8NH$ umsetzt.

7. Verfahren zur Herstellung von Verbindungen der Formel Ib

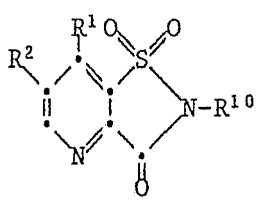

(Ib),

worin $R^1$ und $R^2$ die im Anspruch 1 gegebene Bedeutung haben und $R^{10}$ die im Anspruch 1 für R angegebene Bedeutung hat, aber kein α,α-verzweigter Rest $R^9$ gemäss Anspruch 10 oder H ist, dadurch gekennzeichnet, dass man eine Verbindung der Formel II

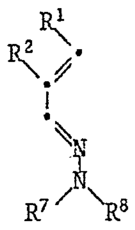

(II),

worin $R^7$ und $R^8$ unabhängig die Bedeutung von $R^3$ und $R^4$ haben, in Gegenwart von Sauerstoff und einem organischen Sulfoxid als Lösungsmittel bei erhöhten Temperaturen mit einer Verbindung der Formel IIIb

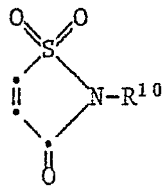

(IIIb),

zu einer Verbindung der Formel VI

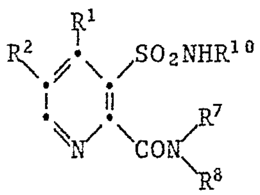

(VI)

umsetzt, und die Verbindung VI unter Abspaltung von $R^7R^8NH$ zu einer Verbindung der Formel Ib cyclisiert.

8. Verfahren gemäss einem der Ansprüche 5 oder 6, dadurch gekennzeichnet, dass es bei einer Temperatur von 50 bis 200° C durchgeführt wird.

9. Verfahren gemäss einem der Ansprüche 5 oder 6, dadurch gekennzeichnet, dass das organische Sulfoxid Dimethylsulfoxid ist.

10. Verfahren gemäss Anspruch 6, dadurch gekennzeichnet, dass die Cyclisierung durch Einwirkung einer starken Säure erfolgt.

11. Verbindungen der Formel IV

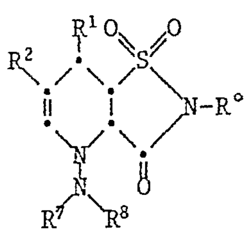

(IV)

worin $R^0$ für lineares oder verzweigtes $C_1$-$C_{20}$-Alkyl, $C_2$-$C_{20}$-Alkenyl oder $C_2$-$C_{20}$-Alkinyl, $C_3$-$C_{10}$-Cycloalkyl, $C_4$-$C_{20}$-Cycloalkylalkyl, $C_4$-$C_{20}$-Alkylcycloalkyl, $C_5$-$C_{20}$-Alkylcycloalkylalkyl, $C_6$-$C_{14}$-Aryl, $C_7$-$C_{20}$-Aralkyl oder -Alkaryl oder $C_8$-$C_{20}$-Alkaralkyl steht, das unsubstituiert oder mit -OH, -CN, Halogen, $C_1$-$C_{12}$-Alkoxy oder Alkylthio, $C_6$-$C_{10}$-Aryloxy oder -thio, $C_7$-$C_{16}$-Aralkyloxy, -Alkaryloxy, -Aralkylthio oder -Alkarylthio, $C_8$-$C_{18}$-Alkaralkyloxy oder -thio, $-NR^3R^4$, $-COOR^5$, $-OCOR^6$, $-CONR^3R^4$ oder $-NR^3COR^6$

substituiert ist, worin $R^3$ und $R^4$ unabhängig voneinander $C_1$-$C_{12}$-Alkyl, $C_5$- oder $C_6$-Cycloalkyl, $C_6$-$C_{10}$-Aryl, $C_7$-$C_{16}$-Aralkyl, $C_8$-$C_{16}$-Alkaralkyl oder $R^3$ und $R^4$ zusammen Tetra- oder Pentamethylen oder 3-Oxapentylen, $R^5$ H, $C_1$-$C_{12}$-Alkyl, Phenyl, Benzyl, Cyclohexyl oder Cyclopentyl und $R^6$ $C_1$-$C_{12}$-Acyl sind,
$R^1$ lineares oder verzweigtes $C_1$-$C_{12}$-Alkyl darstellt, das unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert ist,
$R^2$ $C_1$-$C_{12}$-Alkyl oder -Alkoxy, $C_6$-$C_{10}$-Aryl oder -Aryloxy, $C_7$-$C_{16}$-Aralkyl, -Alkaryl, -Alkaryloxy oder -Aralkyloxy, $C_8$-$C_{16}$-Alkaralkyloxy bedeutet, die unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert sind, und $R_7$ und $R_8$ die für $R_3$ und $R_4$ angegebene Bedeutung haben.

12. Verbindungen der Formel V

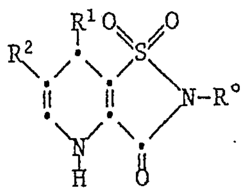

(V),

worin $R^0$ für lineares oder verzweigtes $C_1$-$C_{20}$-Alkyl, $C_2$-$C_{20}$-Alkenyl oder $C_2$-$C_{20}$-Alkinyl, $C_3$-$C_{10}$-Cycloalkyl, $C_4$-$C_{20}$-Cycloalkylalkyl, $C_4$-$C_{20}$-Alkylcycloalkyl, $C_5$-$C_{20}$-Alkylcycloalkylalkyl, $C_6$-$C_{14}$-Aryl, $C_7$-$C_{20}$-Aralkyl oder -Alkaryl oder $C_8$-$C_{20}$-Alkaralykl steht, das unsubstituiert oder mit -OH, -CN, Halogen, $C_1$-$C_{12}$-Alkoxy oder -Alkylthio, $C_6$-$C_{10}$-Aryloxy oder -thio, $C_7$-$C_{16}$-Aralkyloxy, -Alkaryloxy, -Aralkylthio oder -Alkarylthio, $C_8$-$C_{18}$-Alkaralkyloxy oder -thio, $-NR^3R^4$, $-COOR^5$, $-OCOR^6$, $-CONR^3R^4$ oder $-NR^3COR^6$ substituiert ist, worin $R^3$ und $R^4$ unabhängig voneinander $C_1$-$C_{12}$-Alkyl, $C_5$- oder $C_6$-Cycloalkyl, $C_6$-$C_{10}$-Aryl, $C_7$-$C_{16}$-Aralkyl, $C_8$-$C_{16}$-Alkaralkyl oder $R^3$ und $R^4$ zusammen Tetra- oder Pentamethylen oder 3-Oxapentylen, $R^5$ H, $C_1$-$C_{12}$-Alkyl, Phenyl, Benzyl, Cyclohexyl oder Cyclopentyl und $R^6$ $C_1$-$C_{12}$-Acyl sind,
$R^1$ lineares oder verzweigtes $C_1$-$C_{12}$Alkyl darstellt, das unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert ist,
$R^2$ $C_1$-$C_{12}$-Alkyl oder -Alkoxy, $C_6$-$C_{10}$-Aryl oder -Aryloxy, $C_7$-$C_{16}$-Aralkyl, -Alkaryl, -Alkaryloxy oder -Aralkyloxy, $C_8$-$C_{16}$-Alkaralkyloxy bedeutet, die unsubstituiert oder mit Hydroxy, Halogen, Cyano, $C_1$-$C_4$-Alkoxy, Carboxyl, $C_1$-$C_{12}$-Acyloxy oder $C_1$-$C_4$-Alkoxycarbonyl substituiert sind.

Europäisches Patentamt

# EUROPÄISCHER RECHERCHENBERICHT

Nummer der Anmeldung

EP 88 81 0611

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 113 927 (MANUFACTURE DE PRODUITS PHARMACEUTIQUES)<br>* Anspruch 1 *<br>--- | 1 | C 07 D 513/04<br>A 01 N 43/90 |
| A,D | EP-A-0 013 480 (E.I. DU PONT DE NEMOURS & CO.)<br>* Anspruch 1 *<br>----- | 1 | |
| | | | **RECHERCHIERTE SACHGEBIETE (Int. Cl.4)** |
| | | | C 07 D 513/00 |

Der vorliegende Recherchenbericht wurde für alle Patentansprüche erstellt

| Recherchenort | Abschlußdatum der Recherche | Prüfer |
|---|---|---|
| DEN HAAG | 15-12-1988 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument